# EUROPEAN PATENT APPLICATION

(11) **EP 1 386 906 A1**
(43) Date of publication of application: **04.02.2004**
(21) Application number: 03254583.2
(22) Date of filing: 22.07.2003
(51) Int. Cl.: C07C 51/42, B01D 53/56, B01D 53/62, F23G 7/06

(54) **Method for reducing nitrogen oxide emissions in industrial processes**

(30) Priority: 02.08.2002 US 400536 P
(71) Applicant: ROHM AND HAAS COMPANY, Philadelphia, Pennsylvania 19106-2399 (US)
(72) Inventor: Decourcy, Michael Stanley, Houston, Texas 77062 (US); Pugh, Patrick Kevin, Kingwood, Texas 77345 (US)
(74) Representative: Kent, Venetia Katherine

(57) **Abstract**

One embodiment of the invention provides novel methods for reducing the generation of NOx emissions in industrial processes. These methods include the steps of: feeding a reaction feed mixture to a reaction vessel having an inlet and an outlet; producing a reaction product including a hot mixed gas stream in the reaction vessel; producing a cooled mixed gas stream by directing the hot mixed gas stream to a heat exchanger system; separating the cooled mixed gas stream into a cooled crude product stream and a cooled waste stream; producing a preheated waste stream by directing the cooled waste stream to a heat exchanger system; and incinerating the preheated waste stream by directing it to an incinerator. Upon incineration of the preheated waste stream, a decreased amount of NOx emissions is produced when compared to the amount of NOx emissions which would have been produced by incinerating the cooled waste stream.

## Description

### FIELD OF THE INVENTION

This invention relates to methods for reducing unwanted emissions in industrial processes. More specifically, this invention relates to methods for decreasing nitrogen oxides ("NOx") production associated with thermal oxidation operations.

### BACKGROUND

In conventional industrial processes for making products such as acrylic acid from propylene, a reaction feed mixture, typically comprises propylene, oxygen, and optionally water, or nitrogen or other inerts. A two-stage catalytic reactor is typically utilized to oxidize the propylene to acrolein in a first reactive stage, and to then oxidize the acrolein to acrylic acid in a second reactive stage. Conventional two-stage reactors may comprise, for example, a single-shell reactor system (an "SRS"), a tandem reactor system, or a staged-air tandem reactor system, all of which are well known to those skilled in the art.

The output from the catalytic reactor is a hot mixed gas stream comprising acrylic acid, unreacted propylene, nitrogen, and other impurities, such as water, carbon monoxide, carbon dioxide, and acrolein. The hot mixed gas stream passes into a separation step, which serves to separate the acrylic acid from the impurities.

Conventional separation steps typically comprise equipment such as absorbers or extraction columns. The separation step produces at least two streams: a crude product stream comprising primarily acrylic acid, and a waste stream comprising primarily impurities.

The crude product stream is typically further processed to produce product grade acrylic acid or other products, such as acrylic acid esters. The waste stream is typically fed to an incineration step, along with supplemental fuel, such as natural gas, and an oxygen-containing gas stream, such as atmospheric air or oxygen-enriched air. The incineration step typically comprises processing equipment such as thermal oxidizers, incinerators, furnaces, or other combustors suitable for providing high-efficiency destruction of waste streams. In the incineration step, the waste stream is combusted or thermally decomposed to create an effluent stream, comprising inerts - such as water and carbon dioxide - and also thermal NOx.

Examples of conventional processes for producing acrylic acid can be found in U.S. Patent Nos. 5,817,865; 6,166, 248; and 6,350,906.

Thermal NOx is formed when the nitrogen present in atmospheric air is subjected to high temperatures, such as those normally found in conventional combustion and incineration processes. Once released to the environment, thermal NOx is chemically indistinguishable from other forms of NOx.

NOx is usually a combination of nitric oxide and nitrogen dioxide. NOx is an industrial pollutant whose production is regulated by governmental agencies such as the Environmental Protection Agency (EPA) in an effort to set limits on its production. It is, therefore, desirable to minimize the formation of thermal NOx in industrial processes.

Numerous methods and processes have been developed that are directed to reducing NOx emissions produced by industrial processes. These methods include: ammonia injection directly into effluent, chemical scrubbers, modification of furnace burners of an incinerator to reduce NOx production, and catalytic treatment of effluent. These and other previously developed methods can be found in U.S. Patent Nos. 6,348,178 and 6,193,934.

However, these previously developed methods suffer from the disadvantage of treating NOx after it has been produced, or of producing additional unwanted emissions in the treatment process itself. For example, they do not provide a method preventing NOx production, or a method of reducing NOx emissions without adding other combustion sources, chemicals, or limited life components. Therefore, a need exists for a method of reducing NOx emissions by preventing NOx production without introducing additional energy consumption or chemical agents, and utilizes thermal energy existing within an industrial processing facility.

### STATEMENT OF INVENTION

Therefore, one object of the present invention provides novel processes for reducing NOx emissions in industrial processes.

Another object of this invention provides novel acrylic acid production processes which produce, among other things, lower NOx emissions.

These and other objects will be apparent to those skilled in the art after reading the specification and appended claims.

Accordingly, one embodiment of the present invention provides novel processes for reducing the generation of NOx emissions. These processes comprise the steps of: feeding a reaction feed mixture to a reaction vessel having an inlet and an outlet; producing a reaction product comprising a hot mixed gas stream in the reaction vessel; producing a cooled mixed gas stream by directing the hot mixed gas stream to a heat exchanger system; separating the cooled mixed gas stream into a cooled crude product stream and a cooled waste stream; producing a preheated waste stream by directing the cooled waste stream to a heat exchanger system; and incinerating the preheated waste stream by directing it to an incinerator. Upon incineration of the preheated waste stream, a decreased amount of NOx emissions is produced when compared to the amount of NOx emissions which would have been produced by incinerating the cooled waste stream.

Another embodiment of the present invention provides novel processes for producing acrylic acid, wherein the processes comprise a reaction step, a separation step, and an incineration step. The reaction step comprises the step of: heating a feed stock comprising propylene in the presence a catalyst to produce a reaction product comprising acrylic acid and waste products. The separation step comprises the step of: separating the reaction product into an acrylic acid stream and a waste product stream. The incineration step comprises the steps of: producing a preheated waste product stream, and incinerating the preheated waste product stream.
Figure 1 illustrates a schematic of one embodiment of the present invention.
Figure 2 depicts a schematic of an alternative embodiment of the present invention.
Figure 3 illustrates a schematic of yet another alternative embodiment of the present invention.

### DETAILED DESCRIPTION

One embodiment of the present invention relates to methods for reducing NOx emissions. Specifically, by increasing the inlet temperature of a waste gas stream to be incinerated entering an incinerator reduces the amount of energy required for incineration. This, in turn, decreases the incinerator's fuel consumption. Moreover, the increased incinerator inlet temperature, when combined with diminished incinerator energy needs, results in a reduction of the product gases generated during incineration. Since NOx is one of the gases formed by incineration, reduction in overall gas production during incineration reduces the NOx production.

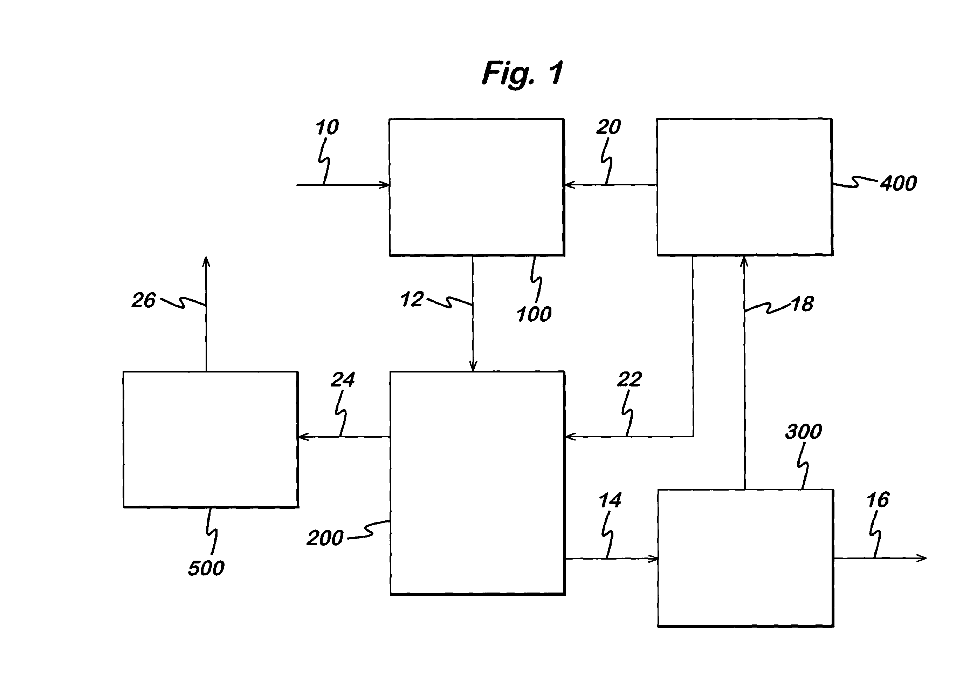

With reference to the drawing herein, a method for reducing NOx content in emissions according to one embodiment of the present invention is shown in Figure 1. Like numbers represent like streams, steps, and elements.

The hot mixed gas stream 12 of Figure 1 enters a heat exchanger section 200 wherein it rejects heat, and exits as a cooled mixed gas stream 14, which then enters separation section 300. A by-product stream 18 exits the separation section 300 and enters into section 400 where it is separated into a recovered product/reactant stream 20 and a waste stream 22. Waste stream 22 is fed into heat exchanger section 200 where it is warmed by the heat recovered from the hot mixed gas stream 12. After being heated, waste stream if fed into incineration section 500 via stream 24. By preheating waste stream 22, less supplemental fuel is required to operate incineration section 500.

The supplemental fuel is typically methane, but can be any hydrocarbon or flammable substance suitable for use within an incinerator or thermal oxidizer. A stream can also be present which provides oxygen to the incineration section 500, generally in the form of atmospheric air. However, the oxygen-containing stream can comprise pure oxygen or other oxygen containing gases. Because less supplemental fuel and oxygen are consumed by virtue of the present invention, less thermal NOx is formed as a result of combustion. Consequently, less thermal NOx is present in the effluent stream 26.

It should be understood that a similar beneficial reduction in thermal NOx generation could be obtained if a different incineration step feed stream were preheated instead of, or in addition to, stream 22. Furthermore, while one preferred method of heating the waste stream 22 is to transfer heat energy from within the process that produces the waste stream 22, another heating medium could be utilized to heat the waste stream 22 as well. The other medium could emanate from fluid streams that already exist within the industrial facility where the process is located, or could be produced specifically for heating the waste stream 22. These streams include hot gases or vapors from within an industrial processing facility, such as steam (at any pressure and temperature), incinerator effluent, or flue gas. The flue gas can be from a reactor, regenerator, or other source.

The hot mixed gas stream 12 shown in Figure 1 contains certain constituents having a melting point above the temperature of the waste stream 22. Consequently, if heat exchangers are employed within the heat exchanger section 200, they should be designed to ensure that the tube wall temperature does not drop below the melting point of any constituent of the hot mixed gas stream 12. If the tube wall temperature is allowed to fall below the melting point of a hot mixed gas stream 12 constituent, the particular constituent could solidify onto the tube wall and ultimately produce undesired fouling of the heat exchanger tubes.

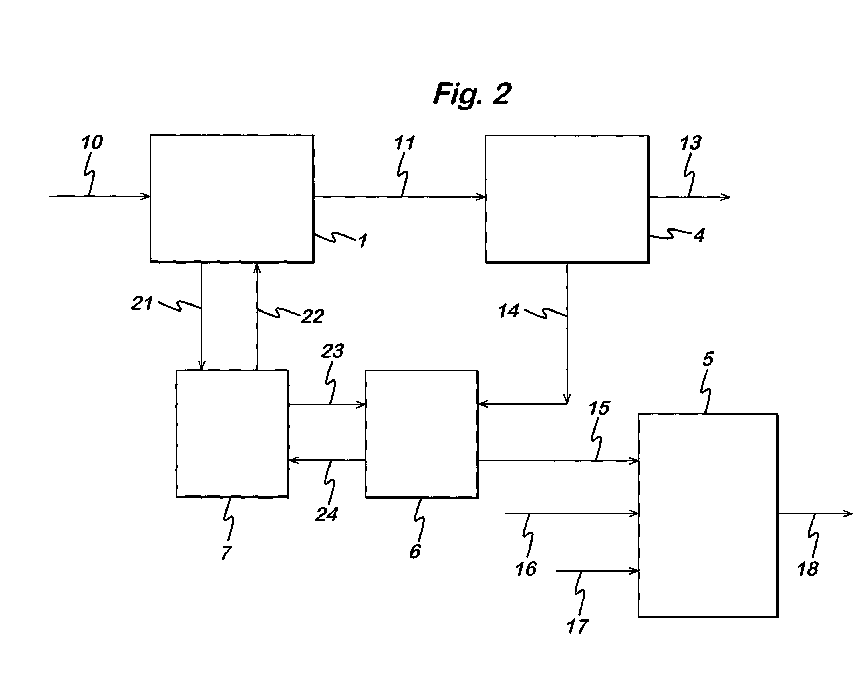

Figure 2 depicts another alternative embodiment of the present invention. Stream 21 represents a hot heat transfer material, such as molten salt or *DOWTHERM®* heat transfer material, which carries heat produced by a conventional catalytic acrylic acid reaction step. Stream 21 is cooled by rejecting heat in the cooling step 7 to the cooling water stream 24. Cooling step 7 can be comprised of one or more heat exchangers. The thermal energy of stream 21 is transferred to the cooling water supplied by cooling water stream 24 in cooling step 7 and, in turn, produces steam 23 that is supplied to heat recovery step 6. Cooled heat transfer material 22 is returned to the reaction step. The steam 23 is then utilized in heat recovery step 6 to preheat waste stream 14 as previously described in the Figure 1.

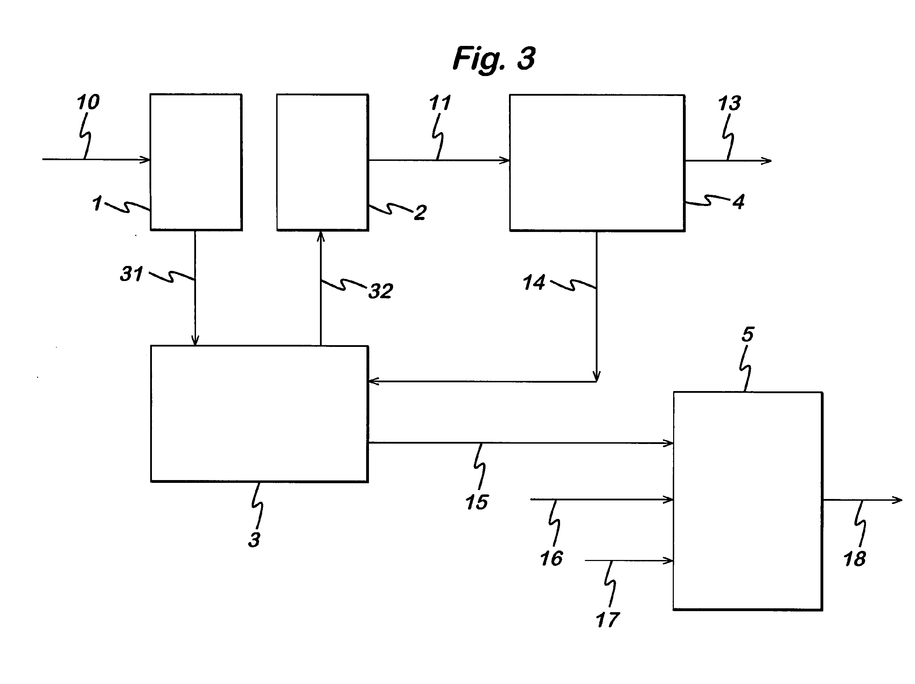

Figure 3 depicts yet another embodiment of the present invention. Here, heat is transferred from an interstage cooler to preheat the waste stream 14. An interstage cooler is typically utilized with a tandem-type acrylic acid reactor. The first stage oxidation reaction takes place in a first reaction zone 1 and produces stream 31 Stream 31 is a hot first reaction stage stream comprising acrolein, which is cooled in the interstage cooler 3 before returning as cooled stream 32. The cooled stream 32 undergoes the second stage reaction in a second reaction zone 2 and is converted to the hot mixed gas stream 11. Recovered heat from the interstage cooler 3 is utilized to preheat the waste stream 14 which, in turn, reduces the NOx produced within the incineration step 5 as previously described herein.

### EXAMPLE

Referring now to the process configuration of Figure 1, an example is provided illustrating the improved and novel features of a specific embodiment of the present invention. This specific embodiment relates to an acrylic acid production process.

Here a feed stream comprising primarily propylene, propane, nitrogen, oxygen, water, and other organics is fed into the reaction section 100 of an acrylic acid production process. Reaction section 100 comprises an SRS reactor.

The feed mixture 10 is derived from three conventional sources: a) a recycle waste stream from an aqueous absorber that is part of a typical acrylic acid separation process step; b) a fresh atmospheric air feed stream from a process air compressor; and c) a chemical grade propylene stream from a supplier comprising of propylene and propane. While it is preferred that the propylene/propane feed be of chemical grade, this stream could also be refinery grade, polymer grade, or various mixes of olefins containing propylene and propane.

The reactor feed mixture 10 is converted in the SRS Reactor to a hot mixed gas stream 12 comprising nitrogen, water, acrylic acid, propane, propylene, carbon dioxide, carbon monoxide, and acrolein. The hot mixed gas stream 12 exits reactor section 100 at a temperature of about 300°C and a pressure of about 100,000 Pa.

The hot mixed product gas stream 12 flows into heat exchanger section 200. Heat exchanger section 200 comprises a shell/tube heat exchanger.

The hot mixed product gas stream 12 flows through tubes of a shell/tube heat exchanger releasing its sensible enthalpy (heat energy). The now cooled mixed product gas 14 exits heat exchanger section 200 at a temperature of about 200°C and a pressure of about 100,000 Pa.

The hot mixed product gas 14 then enters separator section 300. Separator section 300 comprises an aqueous absorber/separator column which produces a crude product stream 16 and a by-product stream 18.

Crude product stream 16 is produced when the aqueous absorber separates acrylic acid and other desired crude products from the inert gases and waste organic compounds. The crude product stream(s) exit this section 300 and may receive further optimal purification. By-product stream 18 exits this section 300 at a temperature of about 70 ° C and a pressure of about 100,000 Pa. By-product stream 18 comprises nitrogen, water, propane, propylene, acrolein, and other organics.

By-product stream is fed into section 400 where it is split into a reactor recycle stream 20 and a waste stream 22. Recycle stream is fed back into reaction section 100. Waste stream 22 is fed into heat exchanger section 200.

To design against the potential solidification problem within the heat exchanger section 200, a low efficiency heat exchanger is preferably used where the hot mixed gas stream passes through the tube side (from top to bottom) and the waste stream passes through the shell side (from bottom to top). Further, the preferred heat exchanger contains a so called "disk and donut" baffle configuration on its shell side that directs flow alternatively from the middle of the tube sheet to the outside of the tube sheet. The disk baffles are disk shaped and coaxially disposed within the heat exchanger, their diameter is roughly the same as the diameter of the tube bundle. The donut baffles are also disk shaped and are coaxially positioned within the heat exchanger. However, the diameters of the donut baffles are substantially equal to the inner diameter of the shell side of the heat exchanger. Each donut baffle contains an aperture or opening at their center, where the opening diameter is smaller than each disk diameter.

The disk and donut baffles are preferably disposed within a plenum such that the shell side flow enters through the nozzle, proceeds downward to the plenum entrance, up through the disk/donut configuration, exits the plenum, and travels between the plenum and the inside of the shell to the exit nozzle. Upon entering the heat exchanger, the waste stream 14 encounters an annulus distributor designed to force the flow around and upward and to release any entrained liquids. The knocked out liquids will flow back to the separation process through drains. This prevents concentrated flows from impinging directly on the lowermost three feet of the heat exchanger tubes, and thus prevents localized subcooling zones on the exchanger tubes.

After passing heat exchanger section 200, the waste stream is heated to a temperature of about 250° C and a pressure of about 100,000 Pa. At this point, the heated waste stream recovers about 4.52x10⁶ J/s (15.45 MMBtu per hour) of sensible heat from the hot mixed gas stream 12. Heated waste stream 24 then passes into incinerator section 500.

The purpose of the incinerator is to destruct and or produce pyrolysis of the volatile organic compounds (VOC's) in the waste in accordance with environmental regulations. A conventional incinerator step feeds atmospheric air, generally natural gas as a fuel, and the preheated waste stream 24 into a firebox. The fuel may also comprise other conventional liquids, gaseous, or solid fuels, either alone or in combination.

Air is a conventional source of oxygen to support combustion, such as atmospheric air, but may be any number of other conventional chemical oxidants including pure oxygen. However, in industrial operations essentially all oxidizing gases contain nitrogen.

The incinerator effluent is typically comprised of nitrogen, carbon dioxide, carbon monoxide, oxygen, VOC's, NOx, and other compounds. When the preheated waste stream 24 enters the incinerator, about 4.52x10⁶ J/s (15.45 MMBtu per hour) less combustion fuel is needed to attain the minimum require firebox destruction temperature as opposed to a conventional non-preheated waste stream. This preheated waste stream 24 will be responsible for about an additional 2.31x10⁶ J (7.26 MMBtu per hour) reduction in firebox fuel requirements due to the reduced combustion air (CA) flow demand.

In present known processes, it is required to burn about 4.52x10⁶ J/s (15.45 MMBtu) of more fuel in the incinerator step than by utilization of the present improved process. The total fuel savings for this scenario using the improved process is about 6.65x10⁶ J/s (22.71 MMBtu per hour) or about 2.11x10¹⁴ J/year (200,000 MMBtu per year). This results in a reduction of incinerator NOx emission by about 8620 Kg/year (19,000lbs per year). Further, the fuel reduction from this scenario would reduce CO production by approximately 7260 Kg/year (16,000 lbs per year).

It should be noted that the heat transfer between the hot mixed gas stream 12 and the waste stream 24 can be accomplished by a single heat exchanger, or multiple heat exchangers. Further, when multiple heat exchangers are implemented, the heat transfer fluid can be a liquid, a vapor, a molten compound, or any combination thereof, such as a two phase mixture and liquid and vapor.

It is important to note that the novel manner thermal energy transfer that is a part of this invention contributes to the improved results achieved by this invention. More specifically, heating the waste stream 14 with thermal energy from within the process (i.e. the reactor overhead) is a more effective and efficient way of reducing NOx emissions from the incinerator effluent stream 26.

Thermal heating obtained from outside the process requires outside heating and, thus, causes some additional NOx production. Additionally, reducing the heat load within the process itself in turn reduces the cooling water duty associated with the separation step. Reducing the cooling water duty eliminates the need for electrical power to operate the cooling water pumps and cooling water tower. This also contributes to overall NOx production.

The present invention described herein, therefore, is well adapted to carry out the objects and attain the ends and advantages mentioned, as well as others inherent therein. While several presently preferred embodiments of the invention have been given for purposes of disclosure, numerous changes in the details of procedures may be made for accomplishing the desired results. For example, the present invention can involve any process stream having thermal variations that includes incineration as a part of the process. Further, process streams from acrilonitrile, methacrylic acid and other similar products may be treated according to this invention. These and other similar modifications will readily suggest themselves to those skilled in the art, and are intended to be encompassed within the spirit of the present invention disclosed herein and the scope of the appended claims.

## Claims

1. A method for reducing the emission of gaseous products into the atmosphere comprising the steps of:
a. producing a reaction product comprising a hot mixed gas stream by feeding a reaction feed mixture through an inlet of a reaction vessel, and passing the reaction product through an outlet of the reaction vessel;
b. producing a cooled mixed gas stream by directing the hot mixed gas stream through a heat exchanger system;
c. separating the cooled mixed gas stream into a cooled crude product stream and a cooled waste stream;
d. producing a preheated waste stream by directing the cooled waste stream through a heat exchanger system; and
e. incinerating the preheated waste stream by directing it into an incinerator.

2. The method of claim 1, wherein the heat exchanger system of step b is the same as the heat exchanger system of step d.

3. The process of claim 1, wherein the cooled crude product comprises acrylic acid.

4. The method of claim 1, wherein separation of the cooled mixed gas stream into the cooled crude product stream and the cooled waste stream comprises passing the cooled mixed gas stream into a separator column.

5. The method of claim 1, wherein the preheated waste stream is incinerated in an incinerator.

6. The method of claim 1, wherein the heat exchanger system of either step b, or step d, or both steps b and d, comprises a shell and tube exchanger configured to ensure that each constituent of the hot mixed gas stream remains above the respective constituent's melting point.

7. The method of claim 6, wherein the shell and tube exchanger comprises a shell portion and a tube portion, and a series of disk-shaped and donut-shaped baffles disposed within the shell and tube exchanger's shell portion, said series of disk-shaped and donut-shaped baffles being configured such that the hot mixed gas alternates across the tubes of said shell and tube exchanger.

8. The method of claim 1, wherein the gaseous emissions comprise at least one of the following: nitrogen dioxide, nitric oxide, and carbon monoxide.

9. The method of claim 1, wherein the preheated waste stream is incinerated through the use of a preheated supplemental fuel.

10. The method of claim 9, wherein the preheated waste stream is incinerated through the use of a preheated oxygen-containing stream.
